# EUROPÄISCHE PATENTSCHRIFT

(11) **EP 0 729 438 B1**
(45) Veröffentlichungstag und Bekanntmachung des Hinweises auf die Patenterteilung: **09.04.1997**
(21) Anmeldenummer: 94931576.6
(22) Anmeldetag: 02.11.1994
(51) Int. Cl.: C01D 17/00, C07C 51/41

(54) **VERFAHREN ZUR HERSTELLUNG EINER CÄSIUM- UND RUBIDIUMSALZLÖSUNG**
METHOD OF PREPARING A CAESIUM AND RUBIDIUM SALT SOLUTION
PROCEDE DE PREPARATION D'UNE SOLUTION DE CESIUM ET DE RUBIDIUM

(30) Priorität: 16.11.1993 DE 4339062
(43) Veröffentlichungstag der Anmeldung: 04.09.1996
(73) Patentinhaber: METALLGESELLSCHAFT AG, 60323 Frankfurt am Main (DE)
(72) Erfinder: HOFMANN, Hartmut, D-65812 Soden (DE); KÖBELE, Klaus, D-63128 Dietzenbach (DE); PRINZ, Horst, D-61169 Friedberg (DE); SCHADE, Klaus, D-65205 Wiesbaden (DE)
(86) Internationale Anmeldenummer: EP9403651
(87) Internationale Veröffentlichungsnummer: WO9513986

(56) Entgegenhaltungen:
- WO-A-94/11303
- US-A- 3 207 571
- US-A- 3 489 509
- THE SOVIET JOURNAL OF NON-FERROUS METALS, Bd.2, Nr.1, 1961 Seiten 57 - 59 Y.G.GOROSHCHENKO ET AL. 'Obtaining cesium carbonate from pollucite-spodumene concentrate' in der Anmeldung erwähnt
- CHEMICAL ABSTRACTS, vol. 73, no. 16, 19. Oktober 1970, Columbus, Ohio, US; abstract no. 79009y, E. B. PANASENKO ET AL. 'Reaction of pollucite with lime water solutions at 220 C'
- CHEMICAL ABSTRACTS, vol. 100, no. 20, 14. Mai 1984, Columbus, Ohio, US; abstract no. 160074p, Y. NAKAMICHI ET AL. 'Hydrothermal extraction of useful elements from various ores'
- CHEMICAL ABSTRACTS, vol. 79, no. 1, 9. Juli 1973, Columbus, Ohio, US; abstract no. 4949v, E.M.SHIPILOVA ET AL. 'Cesium formate' in der Anmeldung erwähnt

## Beschreibung

Die Erfindung betrifft ein Verfahren zur Herstellung einer Cäsium- und Rubidiumsalzlösung mit einer Dichte zwischen 1,6 und 3,3 g/cm³ durch hydrothermalen Aufschluß von uncalciniertem Pollucit und/oder calciniertem Lepidolith im Zeitraum von 0,5 bis 3 Stunden mit einer wäßrigen Ca(OH)₂-Lösung bei einer Aufschlußtemperatur von 200 bis 280°C und einem Druck von 15 bis 65 bar sowie bei einer Suspensionsdichte zwischen 8 und 18 Gew.%, Abtrennung von den unlöslichen Feststoffen, gegebenenfalls Entfernung von Calcium- und Lithium-Ionen durch Begasung mit Kohlendioxid und Abtrennung der ausgefallenen Carbonate aus dem Filtrat des Aufschlusses sowie Bildung der Salze des Cäsiums und Rubidiums durch Zugabe einer Säure oder eines Säureanhydrids bis zu einem pH-Wert von mindestens 6, wobei zum Erreichen der Dichte der Cäsium- und Rubidiumsalzlösung eine Einengung durch Eindampfung nach dem Aufschluß, nach der Abtrennung der ausgefallenen Carbonate und/oder nach der Zugabe der Säure oder des Säureanhydrids vorgenommen wird.

Aus der Zeitschrift "Tsvetnye Metally - The Soviet Journal of Non-Ferrous Metals", Band II, Nr. 5, S. 57-59 (1961) ist eine Methode zum hydrothermalen Aufschluß eines Pollucit-Spodumen-Konzentrates zur Gewinnung von Cäsiumcarbonat bekannt. Die kalzinierten Mineralien werden dabei einem hydrothermalen Aufschluß mit einer wäßrigen Ca(OH)₂-Lösung bei 220°C und 20 atm Druck über einen Zeitraum von 4 Stunden unterzogen, wobei optimale Aufschlußbedingungen bei 3 Mol Ca(OH)₂ je Mol SiO₂ erzielt werden. Es ließen sich 88,3 % des im Mineral enthaltenen Cäsiums gewinnen und durch Umkristallisation des Alumo-Cäsium-Alauns ein Cäsium-Salz mit einer Reinheit > 99 % herstellen. Weiterhin ist aus dem Chemical-Abstracts-Referat 79/4949v (1973) eine Methode zur Umwandlung von Cs₂CO₃ in CsHCO₂ bekannt, wobei das Carbonat mit Ameisensäure in Wasser reagiert.

Weiterhin beschreibt die deutsche Patentanmeldung P 42 37 954.7 ein Verfahren zur Herstellung von Cäsium- und Rubidiumsalzlösungen mit einer Dichte von 1,6 bis 3,3 g/cm³ durch hydrothermalen Aufschluß von cäsium- und rubidiumhaltigen Mineralien, das die eingangs genannten Verfahrensmerkmale aufweist.

Aufgabe der Erfindung ist es, ausgehend von dem angegebenen Stand der Technik ein Verfahren anzugeben, das bei hohen Ausbeuten und einer Suspensionsdichte oberhalb von 8 Gew.% eine Verringerung des Aufmahlgrades des eingesetzten Minerals bei einem geringen Überschuß von Calciumoxid zu Siliciumdioxid gestattet.

Erfindungsgemäß wird die Aufgabe dadurch gelöst, daß uncalcinierter Pollucit und/oder calcinierter Lepidolith mit einer mittleren Korngröße bis zu 0,5 mm in einem Drehrohr-Autoklaven aufgeschlossen wird, wobei das Molverhältnis von SiO₂ zu CaO im Bereich zwischen 1 : 2,5 und 1 : 1,25 liegt.

Die Suspensionsdichte ist als Konzentration des Pollucits und/oder des Lepidoliths sowie des ungelösten CaO bzw. Ca(OH)₂ in Wasser definiert.

Überraschenderweise lassen sich unter den angegebenen Verfahrensbedingungen beim hydrothermalen Aufschluß in einem Drehrohr-Autoklaven in der Regel mehr als 90 Gew.% des im Mineral enthaltenen Cäsiums und Rubidiums gewinnen und kostengünstig in eine Cäsium- und Rubidiumsalzlösung mit einer Dichte zwischen 1,6 und 3,3 g/cm³ umwandeln, wobei sowohl der Aufmahlaufwand durch Einsatz der Mineralien mit einer mittleren Korngröße bis zu 0,5 mm als auch der Überschuß von CaO gegenüber dem im Mineral enthaltenen SiO₂ vergleichsweise gering ist.

Die weitere, vorteilhafte Ausgestaltung des Verfahrens erfolgt in der Weise, wie es im Anspruch 2 dargelegt ist. Diese Ausgestaltung des erfindungsgemäßen Verfahrens gestattet es, Cäsium- und Rubidiumsalzlösungen mit einer Dichte von 1,6 bis 3,3 g/cm³ herzustellen, wobei Aufschlußlösungen erhalten werden, die weniger eingeengt werden müssen und damit das Verfahren kostengünstiger gestalten.

In vorteilhafter Weise werden die Cäsium- und Rubidiumsalzlösungen erhalten, indem man zur Umsetzung der ggf. nach der Abtrennung von Calcium- sowie Lithium-Ionen verbleibenden Aufschlußlösung als Säuren Ameisensäure, Essigsäure, Zitronensäure, Chlorwasserstoffsäure, Bromwasserstoffsäure oder Schwefelsäure oder die Säureanhydride Kohlenmonoxid, Molybdäntrioxid oder Wolframtrioxid verwendet.

Die Dichte der Cäsium- und Rubidiumsalzlösung ist über weite Bereiche in vorteilhafter Weise dadurch variierbar, daß man gesättigte Lösungen eines Alkali- oder Erdalkalisalzes zumischt, wobei die Anionen beider Salzlösungen gleich sind.

Insbesondere werden die verfahrensgemäß hergestellten Cäsium- und Rubidiumformiatlösungen mit gesättigten Kaliumformiatlösungen zur Einstellung einer Dichte von 1,6 bis 2,26 g/ml gemischt, und verfahrensgemäß hergestellte Cäsium- und Rubidiumbromidlösungen werden mit gesättigten Calciumbromidlösungen zu Salzlösungen mit Dichten zwischen 1,68 und 1,80 g/ml gemischt.

Weiterhin erweist es sich für das Gesamtverfahren als vorteilhaft, die abgetrennten Carbonate zur Lithium-Herstellung und den nach dem hydrothermalen Aufschluß zurückbleibenden unlöslichen Feststoff als Zuschlagstoff zu Zementrohmehlen zu verwenden. Dadurch wird das vorgeschlagene Verfahren nahezu abproduktfrei.

Die Erfindung soll anhand der nachfolgenden Beispiele näher erläutert werden.

| Versuchsmaterial | |
|---|---|
| Gehalt an | Pollucit Original Gew.% |
| Cs | 23,5 |
| Rb | 0,97 |
| Al | 8,9 |
| Na | 1,07 |
| K | 1,09 |
| Li | 0,30 |
| Ca | 0,08 |
| SiO₂ | 51,6 |

Die nachfolgenden Versuche wurden mit dem unkalzinierten Pollucit durchgeführt.

### Beispiel 1

In einem Anschlämmbehälter wird eine 12 Gew.%ige Suspension aus Pollucit, Calciumhydroxid und Wasser hergestellt und vorgewärmt. Das Mol-Verhältnis von SiO₂ zu CaO beträgt 1 : 1,4. Die Ansatzgröße beläuft sich auf 8 m³. Mit dieser Suspension wird ein Drehrohr-Autoklav beschickt. Der Drehrohr-Autoklav besteht aus einem horizontal gelagerten, zylindrischen Druckbehälter mit einem Gesamtvolumen von ca. 13 m³ und einem Arbeitsvolumen von ca. 9 m³. Der Drehrohr-Autoklav wird über einen Zahnantrieb in Rotation versetzt, wobei zwei Geschwindigkeiten (4 bzw. 7 Umdrehungen pro Minute) gefahren werden können. Die Beheizung erfolgt durch direktes Einblasen von Dampf in die Suspension. Der Aufschluß des Pollucit erfolgt unter Rotation bei einer Temperatur von ca. 220°C und einem Druck von 21 bis 23 bar über 1,5 Stunden. Nach Ende der Reaktionszeit wird entspannt und mit Hilfe des Restdruckes die Suspension in die Filtrationsvorlage gedrückt. Der Autoklav wird mit Wasser bei ca. 150°C nachgewaschen und das Waschwasser ebenfalls in die Filtrationsvorlage gedrückt. Die Abtrennung der Aufschlußlösung von den unlöslichen Feststoffen erfolgt in einem Trommelfilter. Der Filterkuchen wird anschließend mit Wasser wieder angemaischt und Hochdruck-Filterkerzen zugeführt. Die erhaltene Suspension wird bei Drücken bis 150 bar in den Hochdruck-Filterkerzen entwässert. Die erzielten Restfeuchten liegen kleiner als 30 %. Die aus Filtrat und Waschwasser bestehende klare Lösung wird eingedampft. Während der fortschreitenden Wasserverdampfung kommt es zur Ausfällung von gelösten Feststoffen. Nach Einengung auf ca. 15 % des Ausgangsvolumens wird in die verbleibende Suspension Kohlendioxid eingeblasen, um Calcium- und Lithium-Ionen als Carbonat zu fällen. Anschließend wird über eine Filternutsche klarfiltriert. Zu dem Filtrat wird Ameisensäure dosiert, bis ein pH-Wert von 6 erreicht ist. Die in Tabelle 1 dargestellten Ergebnisse wurden mit einem Pollucit mit einer mittleren Korngröße von 0,01 mm erzielt.

### Beispiel 2

Der hydrothermale Aufschluß wird gemäß Beispiel 1 durchgeführt mit dem Unterschied, daß der Pollucit eine mittlere Korngröße von 0,2 mm hat. Die Ergebnisse dieses Aufschlusses sind in Tabelle 2 dargestellt.

### Beispiel 3

Der hydrothermale Aufschluß wird gemäß Beispiel 1 durchgeführt mit dem Unterschied, daß jeweils das Waschfiltrat der unlöslichen Feststoffe zum Anmaischen des nachfolgenden Aufschlusses eingesetzt wird. Die Ergebnisse sind in Tabelle 3 dargestellt. In der Tabelle 3 bedeuten Spalte 2 die Menge des Cäsiums im jeweils frisch eingesetzten Mineral und Spalte 3 die Menge des Cäsiums im Waschfiltrat des vorhergehenden Aufschlusses. Spalte 4 gibt die Gesamtmenge aus Ur- und Waschfiltrat an, Spalte 6 gibt die Gewichtsprozente und Spalte 7 den absoluten Gehalt an Cäsium in dem aus Ur- und Waschfiltrat bestehenden Aufschlußfiltrat an. In Spalte 8 ist die prozentuale Ausbeute an Cäsium bezogen auf den im Mineral enthaltenen Gehalt angegeben.

### Beispiel 4

Der hydrothermale Aufschluß wird analog Beispiel 3 durchgeführt mit dem Unterschied, daß ein Pollucit mit einer mittleren Korngröße von 0,2 mm eingesetzt wird. Die Ergebnisse sind in Tabelle 4 zusammengefaßt, wobei die Spalten analoge Bedeutung wie in Tabelle 3 haben.

### Beispiel 5

Cäsium- und rubidiumhaltige Salzlösungen mit einer Dichte von 1,6 bis 2,26 g/ml werden erhalten, indem man die erfindungsgemäß hergestellte Cäsium-, Rubidiumformiatlösung gemäß Tabelle 5 mit gesättigter Kaliumformiatlösung mischt.

### Beispiel 6

Cäsium- und rubidiumhaltige Salzlösungen mit einer Dichte von 1,68 bis 1,80 g/ml werden erhalten, indem man die erfindungsgemäß hergestellten Cäsium-, Rubidiumbromidlösungen gemäß Tabelle 6 mit gesättigter Calciumbromidlösung mischt.

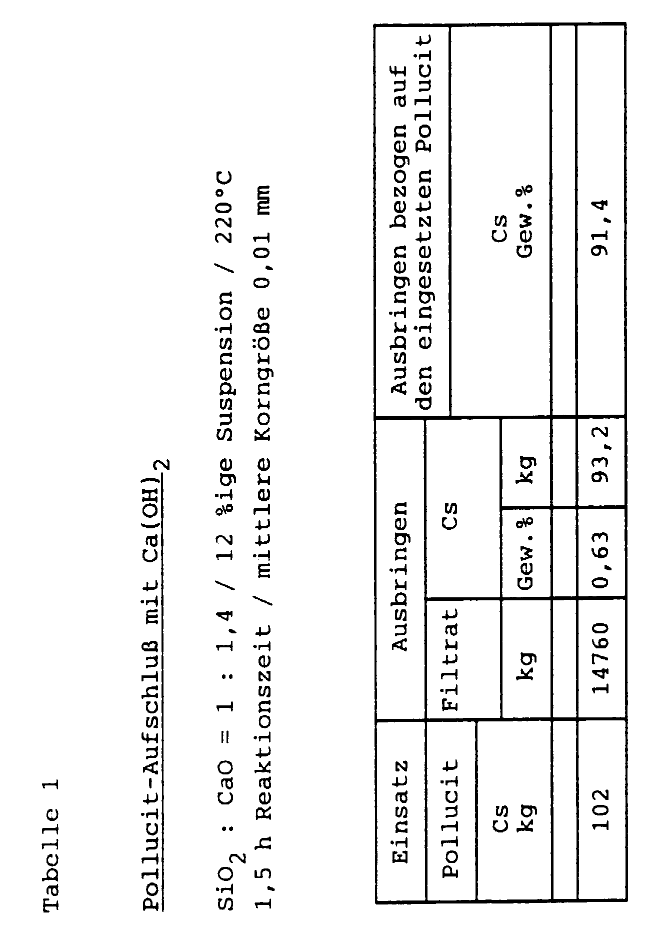

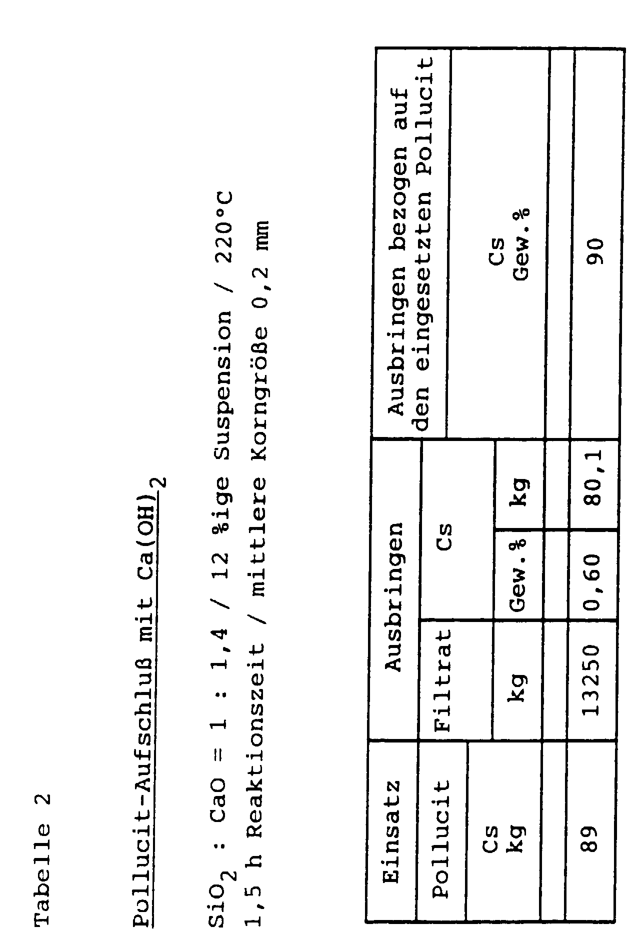

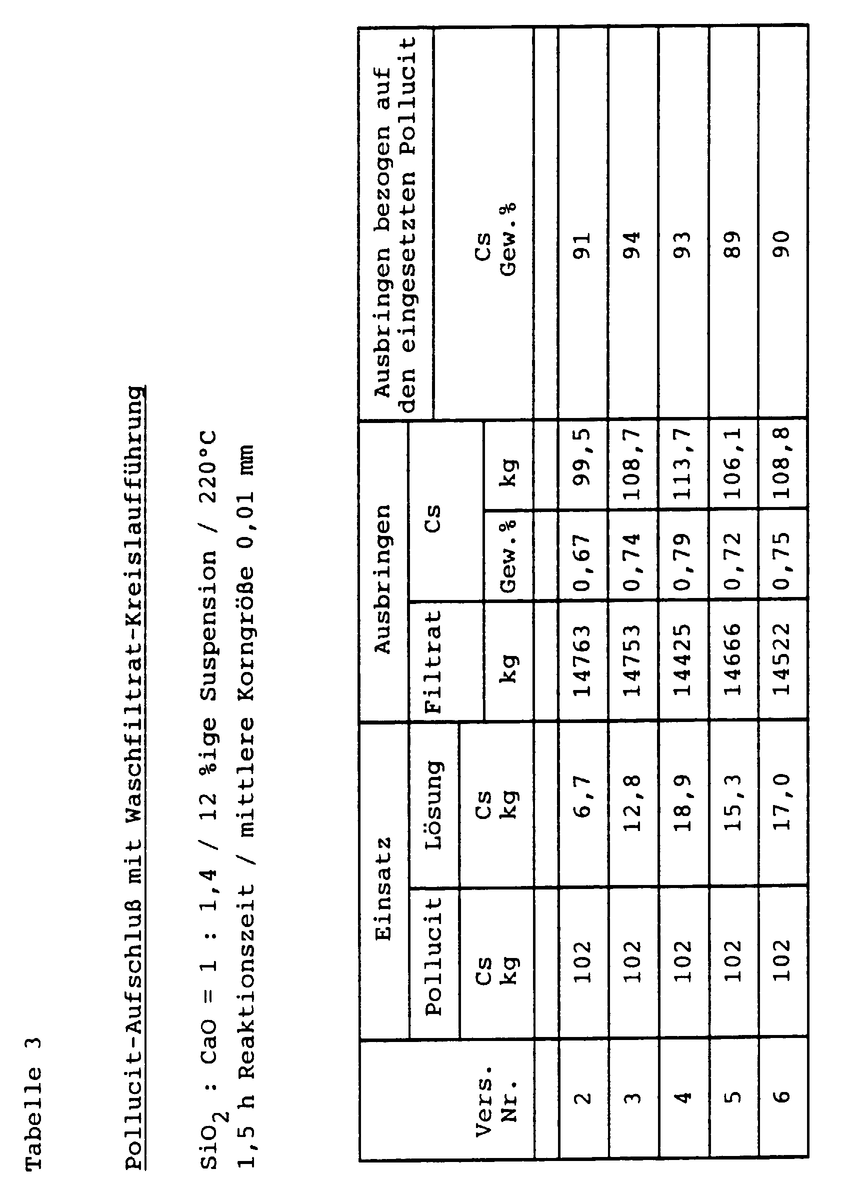

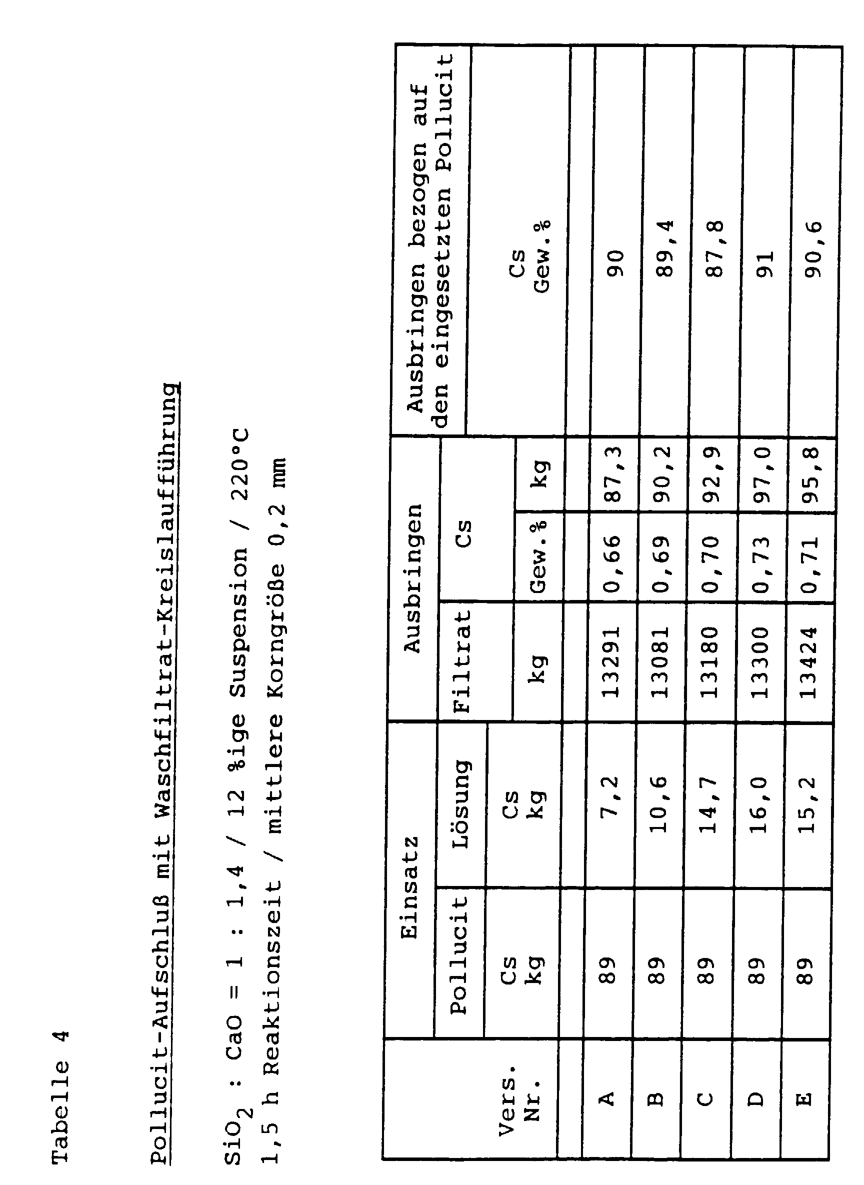

**Tabelle 5**

| Mischung einer HCOOCs/Rb-Lösung, hergestellt aus Pollucit mit einer bei 20°C gesättigten HCOOK-Lösung | | |
|---|---|---|
| Vol.% HCOOCs/Rb : HCOOK | Gew.% HCOOCs/Rb : HCOOK | Dichte bei 20°C g/ml |
| 100 : - | 100 : - | 2,264 |
| 90 : 10 | 92,9 : 7,1 | 2,194 |
| 80 : 20 | 85,2 : 14,8 | 2,125 |
| 70 : 30 | 77,1 : 22,9 | 2,055 |
| 60 : 40 | 68,4 : 31,6 | 1,986 |
| 50 : 50 | 59,1 : 40,9 | 1,916 |
| 40 : 60 | 49,0 : 51,0 | 1,846 |
| 30 : 70 | 38,2 : 61,8 | 1,777 |
| 20 : 80 | 26,5 : 73,5 | 1,707 |
| 10 : 90 | 13,8 : 86,2 | 1,638 |
| - : 100 | - : 100 | 1,568 |

**Tabelle 6**

| Mischung einer Cs/RbBr-Lösung mit einer gesättigten CaBr₂-Lösung | | |
|---|---|---|
| Vol.% Cs/RbBr : CaBr₂ | Gew.% Cs/RbBr : CaBr₂ | Dichte bei 20°C g/ml |
| 100 : - | 100 : - | 1,682 |
| 90 : 10 | 89,3 : 10,7 | 1,695 |
| 80 : 20 | 78,8 : 21,2 | 1,708 |
| 70 : 30 | 68,4 : 31,6 | 1,721 |
| 60 : 40 | 58,2 : 41,8 | 1,734 |
| 50 : 50 | 48,1 : 51,9 | 1,747 |
| 40 : 60 | 38,2 : 61,8 | 1,760 |
| 30 : 70 | 28,5 : 71,5 | 1,773 |
| 20 : 80 | 18,8 : 81,2 | 1,786 |
| 10 : 90 | 9,3 : 90,7 | 1,799 |
| - : 100 | - : 100 | 1,812 |

## Patentansprüche

1. Verfahren zur Herstellung einer Cäsium- und Rubidiumsalzlösung mit einer Dichte zwischen 1,6 und 3,3 g/cm³ durch hydrothermalen Aufschluß von uncalciniertem Pollucit und/oder calciniertem Lepidolith im Zeitraum von 0,5 bis 3 Stunden mit einer wäßrigen Ca(OH)₂-Lösung bei einer Aufschlußtemperatur von 200 bis 280°C und einem Druck von 15 bis 65 bar sowie bei einer Suspensionsdichte zwischen 8 und 18 Gew.%, Abtrennung von den unlöslichen Feststoffen, gegebenenfalls Entfernung von Calcium- und Lithium-Ionen durch Begasung mit Kohlendioxid und Abtrennung der ausgefallenen Carbonate aus dem Filtrat des Aufschlusses sowie Bildung der Salze des Cäsiums und Rubidiums durch Zugabe einer Säure oder eines Säureanhydrids bis zu einem pH-Wert von mindestens 6, wobei zum Erreichen der Dichte der Cäsium- und Rubidiumsalzlösung eine Einengung durch Eindampfung nach dem Aufschluß, nach der Abtrennung der ausgefallenen Carbonate und/oder nach der Zugabe der Säure oder des Säureanhydrids vorgenommen wird, dadurch gekennzeichnet, daß uncalcinierter Pollucit und/oder calcinierter Lepidolith mit einer mittleren Korngröße bis zu 0,5 mm in einem Drehrohr-Autoklaven aufgeschlossen wird, wobei das Molverhältnis von SiO₂ zu CaO im Bereich zwischen 1 : 2,5 und 1 : 1,25 liegt.

2. Verfahren nach Anspruch 1, dadurch gekennzeichnet, daß das nach der Abtrennung der Carbonate erhaltene Urfiltrat und/oder Waschfiltrat als Anmaischflüssigkeit für den nächsten Aufschluß eingesetzt wird.

3. Verfahren nach Anspruch 2, dadurch gekennzeichnet, daß der hydrothermale Aufschluß bei einem Mol-Verhältnis von SiO₂ zu CaO von ca. 1 : 1,4 und einer Suspensionsdichte von ca. 15 Gew.% vorgenommen wird.

4. Verfahren nach einem oder mehreren der Ansprüche 1 bis 3, dadurch gekennzeichnet, daß man der Cäsium- und Rubidiumsalzlösung eine gesättigte Lösung eines Alkali- oder Erdalkalisalzes zumischt, wobei die Anionen beider Salzlösungen gleich sind.

## Claims

1. A method for producing a caesium and rubidium salt solution having a density of between 1.6 and 3.3 g/cm³ by hydrothermal decomposition of uncalcined pollucite and/or calcined lepidolite over a period of 0.5 to 3 hours with an aqueous Ca(OH)₂ solution at a decomposition temperature of 200 to 280°C and a pressure of 15 to 65 bar and at a suspension density of between 8 and 18% by weight, separation of the insoluble solids, optionally removal of calcium and lithium ions by gassing with carbon dioxide and separation of the resulting carbonates from the filtrate of the decomposition and also formation of the salts of caesium and rubidium by the addition of an acid or an acid anhydride up to a pH value of at least 6, with reduction by evaporation in order to reach the density of the caesium and rubidium salt solution being effected after decomposition, after separation of the precipitated carbonates and/or after the addition of the acid or of the acid anhydride, characterised in that uncalcined pollucite and/or calcined lepidolite with an average grain size of up to 0.5 mm is decomposed in a rotary-tube autoclave, the molar ratio of SiO₂ to CaO being in the range between 1 : 2.5 and 1 : 1.25.

2. A method according to Claim 1, characterised in that the original filtrate and/or washing filtrate obtained after the separation of the carbonates is used as a slurrying liquid for the next decomposition.

3. A method according to Claim 2, characterised in that the hydrothermal decomposition is effected at a molar ratio of SiO₂ to CaO of approximately 1 : 1.4 and at a suspension density of approximately 15% by weight.

4. A method according to one or more of Claims 1 to 3, characterised in that a saturated solution of an alkali or alkaline earth salt is admixed to the caesium and rubidium salt solution, the anions of both salt solutions being identical.

## Revendications

1. Procédé de préparation d'une solution de césium et de rubidium ayant une masse volumique comprise entre 1,6 et 3,3 g/cm³, par attaque hydrothermique de pollucite non calcinée et/ou de lépidolite calcinée en un laps de temps de 0,5 à 3 heures par une solution aqueuse de Ca(OH)₂ à une température d'attaque de 200 à 280°C et sous une pression de 15 à 65 bar ainsi que pour une densité de suspension comprise entre 8 et 18% en poids par séparation des matières solides insolubles, par élimination éventuellement des ions calcium et des ions lithium, par barbotage de dioxyde de carbone et par séparation des carbonates précipités du filtrat de l'attaque, ainsi que par formation des sels du césium et du rubidium par addition d'un acide ou d'un anhydride d'acide jusqu'à un pH d'au moins 6, une concentration par évaporation en vue d'obtenir la masse volumique de la solution de sels de césium et de rubidium étant effectué après l'attaque après la séparation des carbonates précipités et/ou après l'addition de l'acide ou de l'anhydride d'acide, caractérisé en ce qu'il consiste à attaquer de la pollucite non calcinée ou de la lépidolite calcinée ayant une granulométrie moyenne allant jusqu'à 0,5 mm dans un autoclave à tube rotatif, le rapport molaire de SiO₂ à CaO étant compris entre 1 : 2,5 et 1 : 1,25.

2. Procédé suivant la revendication 1, caractérisé en ce qu'il consiste à utiliser le filtrat d'origine obtenu après la séparation des carbonates et/ou le filtrat de lavage comme liquide de trempage pour l'attaque immédiatement suivante.

3. Procédé suivant la revendication 2, caractérisé en ce qu'il consiste à effectuer l'attaque hydrothermique à un rapport molaire de SiO₂ à CaO de 1 : 1,4 environ et à une densité de la suspension de 15 % en poids.

4. Procédé suivant une ou plusieurs des revendications 1 à 3, caractérisé en ce qu'il consiste à mélanger à la solution de sels de césium et de rubidium une solution saturée d'un sel de métal alcalin ou d'un sel de métal alcalino-terreux, les anions des deux solutions de sels étant les mêmes.
